# EUROPEAN PATENT SPECIFICATION

(11) **EP 2 793 913 B1**
(45) Date of publication and mention of the grant of the patent: **21.06.2023**
(21) Application number: 12859136.9
(22) Date of filing: 02.11.2012
(51) Int. Cl.: A61K 35/14, A61K 38/17, A61P 9/00, A61K 45/06

(54) **DOSAGE REGIME FOR APOLIPOPROTEIN FORMULATIONS**
DOSIERUNGSSCHEMA FÜR APOLIPOPROTEINFORMULIERUNGEN
RÉGIME POSOLOGIQUE POUR DES FORMULATIONS D'APOLIPOPROTÉINE

(30) Priority: 21.12.2011 AU 2011905368
(43) Date of publication of application: 29.10.2014
(73) Proprietor: CSL Limited, Melbourne, VIC 3000 (AU)
(72) Inventor: RAYNER, Craig, South Yarra, Victoria 3141 (AU)
(74) Representative: Carpmaels & Ransford LLP
(86) International application number: PCT/AU2012/001345
(87) International publication number: WO 2013/090978

(56) References cited:
- WO-A2-02/38609
- WO-A2-03/026492
- WO-A2-03/096983
- WO-A2-2004/010939
- WO-A2-2005/041866
- WO-A2-2007/023476
- JP-A- S61 152 632
- US-A1- 2009 297 577
- TARDIF J C ET AL: "Effects of reconstituted high-density lipoprotein infusions on coronary atherosclerosis - A randomized controlled trial", JAMA THE JOURNAL OF THE AMERICAN MEDICAL ASSOCIATION, AMERICAN MEDICAL ASSOCIATION, US, vol. 297, no. 15, 18 April 2007 (2007-04-18), pages 1675-1682, XP002720081, ISSN: 0098-7484, DOI: 10.1001/JAMA.297.15.JPC70004 [retrieved on 2007-03-26]

## Description

### TECHNICAL FIELD

THIS INVENTION relates to fixed dosing of apolipoprotein formulations. More particularly, this invention relates to the delivery of fixed dosages of apolipoprotein formulations, that is, in an amount independent of patient body weight.

### BACKGROUND

High density lipoprotein (HDL) is a class of heterogeneous lipoproteins containing lipid and protein characterized by high density (>1.063 g/mL) and small size (Stoke's diameter = 5 to 17 nm). The various HDL subclasses vary in quantitative and qualitative content of lipids, apolipoproteins, enzymes, and lipid transfer proteins, resulting in differences in shape, density, size, charge, and antigenicity. Apolipoprotein A-I (Apo-AI) is the predominant HDL protein, although other apolipoproteins such as Apo-AII and Apo-V may be present.

Epidemiological and clinical studies have established an inverse association between levels of high-density lipoprotein cholesterol (HDL-C) and risk of cardiovascular disease (reviewed in Assmann et al., 2004, Circulation 109 III-8). More particularly, clinical administration of apolipoprotein formulations such as in the form of reconstituted HDL (rHDL) formulations have been shown to confer beneficial effects to hypercholesterolemic patients suffering from recent acute coronary syndromes (ACS).

Dosages of apolipoprotein formulations, such as rHDL formulations, are typically calculated according to the body weight of the patient or individual to whom the formulation is administered. This approach to dosage is based on the assumption that there is a direct correlation between a person's body weight and their ability to distribute and eliminate the apolipoprotein formulation. Thus the expectation is that body weight based dosing of the apolipoprotein formulation would result in each patient receiving the same exposure to the apolipoprotein with minimal variation between patients of the same or different body weights.

### SUMMARY

The present inventors have surprisingly discovered that typical dosages of apolipoprotein formulations, such as reconstituted HDL (rHDL) formulations particularly when adjusted or calculated according to patient body weight, display considerable variability between patients in terms of exposure to the apolipoprotein (such as apoA-I) administered in the formulation. More particularly, it has been shown by the inventors that inter-patient. variability in exposure to apolipoprotein when dosing patients based on their body weight is greater than that observed with a fixed dosing approach.

It is an object of the invention to provide an apolipoprotein formulation in a dosage which alleviates or avoids one or more of the deficiencies of prior art apolipoprotein formulations.

It is a preferred object of the invention to provide an apolipoprotein formulation at a dosage that is efficacious in the prophylactic and/or therapeutic treatment of diseases or conditions including, but not limited to cardiovascular disease.

It is another preferred object of the invention to provide an apolipoprotein formulation at a dosage which displays relatively reduced inter-patient variability as measured by patient exposure to apolipoprotein components of the apolipoprotein formulation.

In a first aspect, described herein is a method of prophylactically or therapeutically treating a disease, disorder or condition in a human including the step of administering to the human a fixed dose apolipoprotein formulation, to thereby treat said disease, disorder or condition in the human.

In a second aspect, the invention provides a fixed dosage apolipoprotein formulation for use in prophylactically or therapeutically treating a disease, disorder or condition in a human.

In a third aspect, the invention provides a fixed dosage apolipoprotein formulation comprising an apolipoprotein or a fragment thereof, at a therapeutically effective fixed dose.

Suitably, the fixed dosage apolipoprotein formulation is therapeutically effective upon administration to a human of any body weight, or of any body weight in a body weight range.

Suitably, the fixed dosage apolipoprotein formulation displays relatively reduced inter-patient variability in exposure of the apolipoprotein of the formulation compared to that which would be observed or associated with a weight-adjusted dosage regime.

In a fourth aspect, described herein is a method of producing a fixed dosage apolipoprotein formulation comprising an apolipoprotein or a fragment thereof, including the step of producing the apolipoprotein formulation at a fixed dosage which is therapeutically effective.

Suitably, the method includes the step of determining a fixed dosage of the apolipoprotein formulation that is therapeutically effective upon administration to a human of any body weight or of any body weight in a body weight range. Preferably, the fixed dosage is determined as that at which the apolipoprotein formulation displays relatively reduced inter-patient variability in exposure over a range of patient body weights to the apolipoprotein constituent of the apolipoprotein formulation compared to that which would be observed or associated with weight-adjusted dosage administered to patients over the same weight range.

In a fifth aspect, described herein is an apolipoprotein formulation produced according to the method of the fourth aspect for prophylactically or therapeutically treating a disease, disorder or condition in a human.

In a sixth aspect, described herein is a fixed dosage, apolipoprotein kit comprising one or more unit dosages of a fixed dosage apolipoprotein formulation according to the second or third aspect, or produced according to the method of the fourth aspect; and one or more other kit components.

Preferably, the disease, disorder or condition includes cardiovascular disease, hypercholesterolaemia or hypocholesterolaemia, inclusive of acute coronary syndrome (ACS), atherosclerosis, unstable angina pectoris and myocardial infarction.

In a preferred form the apolipoprotein is Apo-A1.

Suitably, the apolipoprotein formulation is a reconstituted high density lipoprotein (rHDL) formulation comprising an apolipoprotein, a lipid and, optionally, a detergent.

Throughout this specification, unless the context requires otherwise, the words "comprise", "comprises" and "comprising" will be understood to imply the inclusion of a stated integer or group of integers but not the exclusion of any other integer or group of integers.

### BRIEF DESCRIPTION OF THE FIGURES

Reference is made to the following Figures which assist in understanding non-limiting embodiments of the invention described in detail hereinafter wherein:

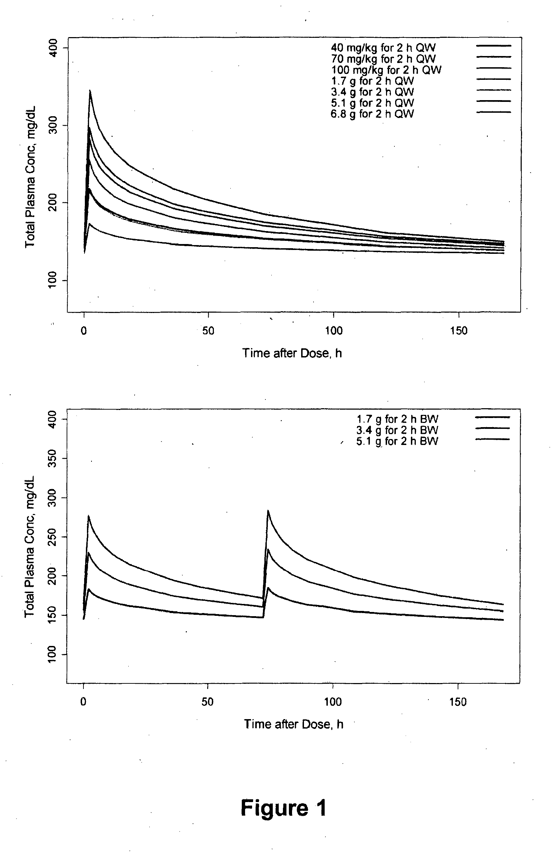

Figure 1 shows median simulated ApoA-I plasma concentration vs. time profiles by dosing regimen (2 h infusion duration only) during week 4 of dose administration;

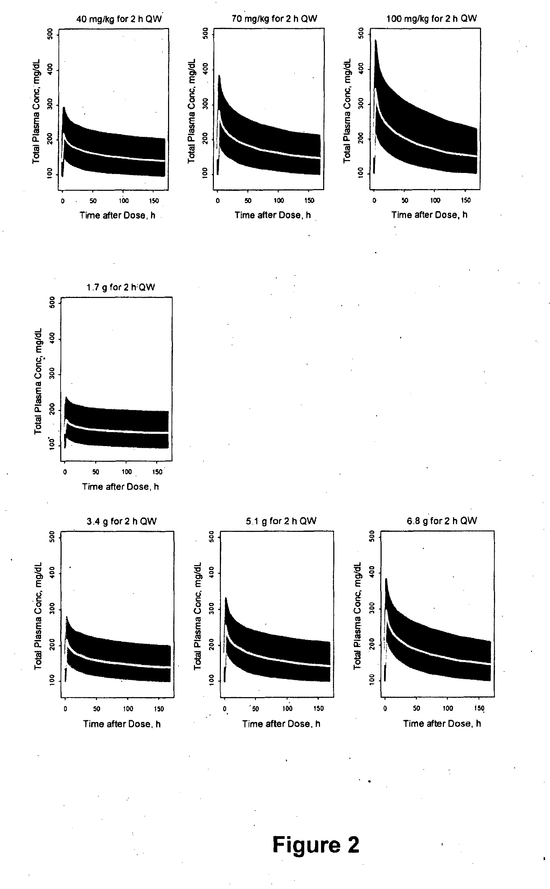

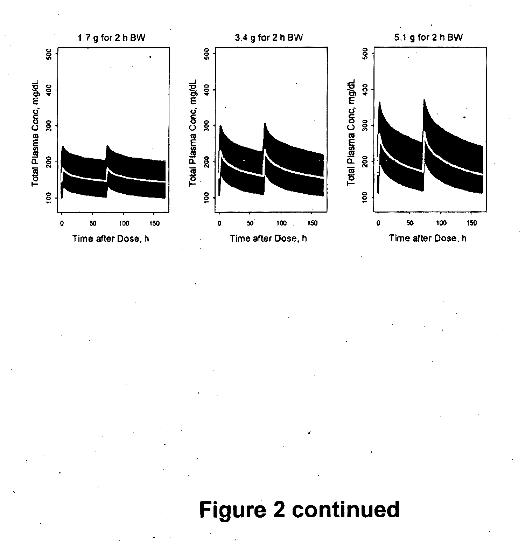

Figure 2 shows projected distributions of total ApoA-I plasma concentration vs. time profiles by dosing regimen (2 h infusion duration only) during week 4 of dose administration. Line represents median profile, shaded region represents 95% prediction interval (PI);

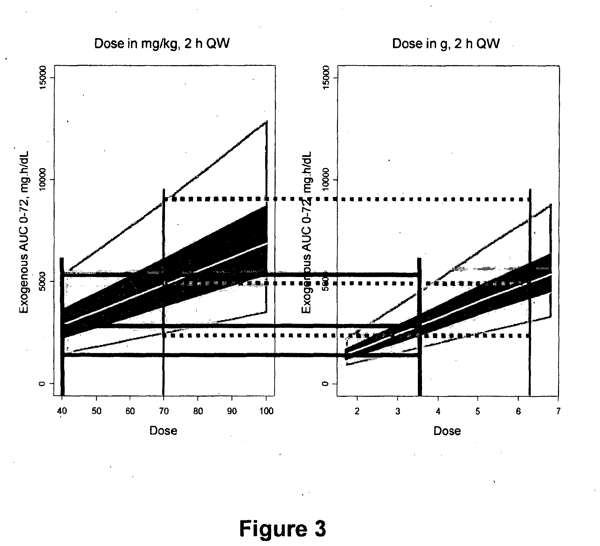

Figure 3 shows distributions of exogenous ApoA-I AUC₀₋₇₂ for the last dose of weekly regimens infused over 2 h. White line represents the median response, dark blue shaded area represents the inter-quartile range and light blue shaded area represents the 95% PI for 100 simulations. Outer solid red horizontal lines show the broader width of the exposure bands for the 40 mg/kg dose in the left panel relative to those for the equivalent fixed dose (3.6 g) in the right panel. The inner solid red line joins the median exposure for those doses. Dashed gray lines show the comparative exposures for the 70 mg/kg dose in the left panel and the equivalent fixed dose (6.3 g) in the right panel;

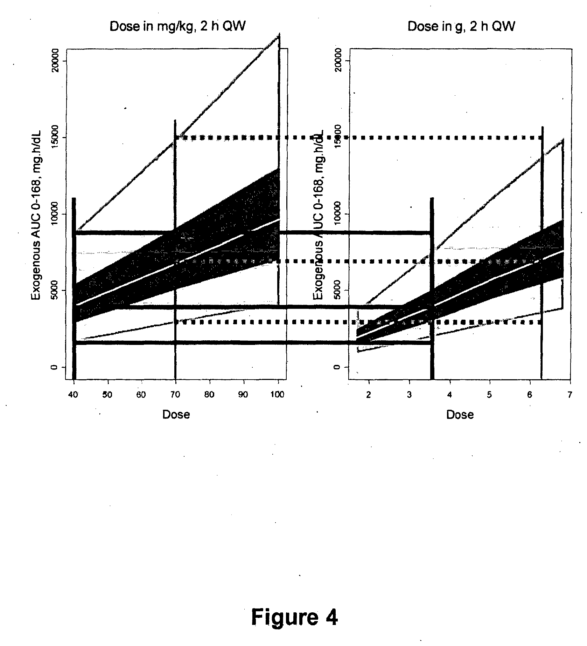

Figure 4 shows distributions of exogenous ApoA-I AUC₀₋₁₆₈ for the last dose of weekly regimens infused over 2 h. White line represents the median response, dark blue shaded area represents the inter-quartile range and light blue shaded area represents the 95% PI for 100 simulations. Outer solid red horizontal lines show the broader width of the exposure bands for the 40 mg/kg dose in the left panel relative to those for the equivalent fixed dose (3.6 g) in the right panel. The inner solid red line joins the median exposure for those doses. Dashed gray
   lines show the comparative exposures for the 70 mg/kg dose in the left panel and the equivalent fixed dose (6.3 g) in the right panel;

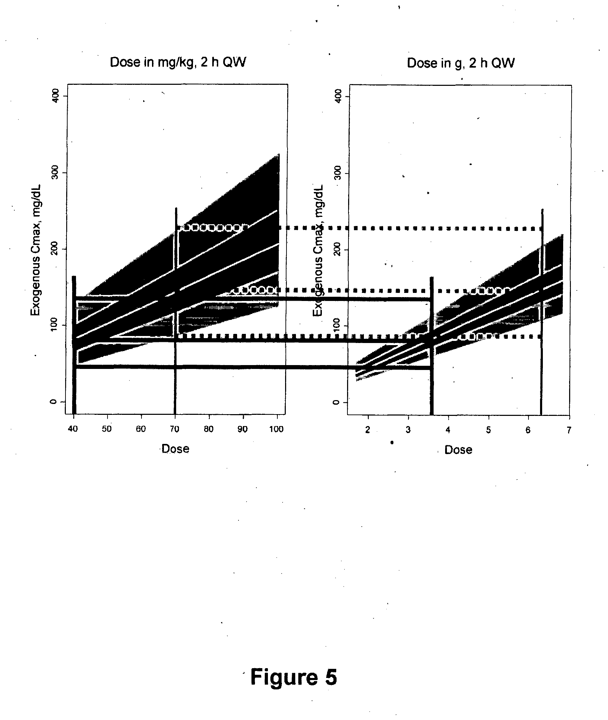

Figure 5 shows distributions of exogenous ApoA-I Cₘₐₓ for the last dose of weekly regimens infused over 2 h. White line represents the median response, dark blue shaded area represents the inter-quartile range and light blue shaded area represents the 95% PI for 100 simulations. Outer solid red horizontal lines show the broader width of the exposure bands for the 40 mg/kg dose in the left panel relative to those for the equivalent fixed dose (3.6 g) in the right panel. The inner solid red line joins the median exposure for those doses. Dashed gray lines show the comparative exposures for the 70 mg/kg dose in the left panel and the equivalent fixed dose (6.3 g) in the right panel;

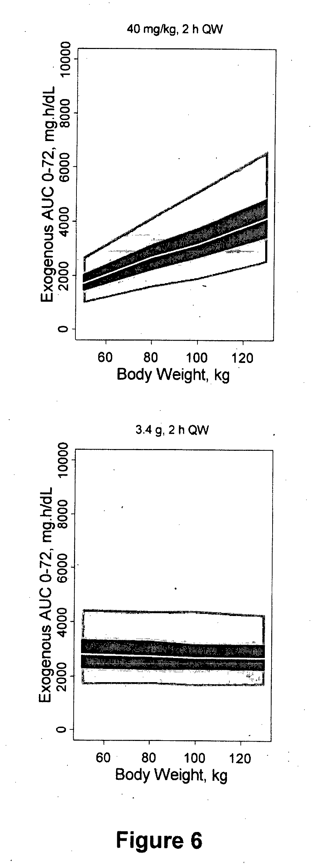

Figure 6 shows the relationship between exogenous ApoA-I AUC₀₋₇₂ (for the last dose of weekly regimens infused over 2 h) between fixed dosing and body weight dosing. White line represents the median response, dark blue shaded area represents the inter-quartile range and light blue shaded area represents the 95% PI for 100 simulations;

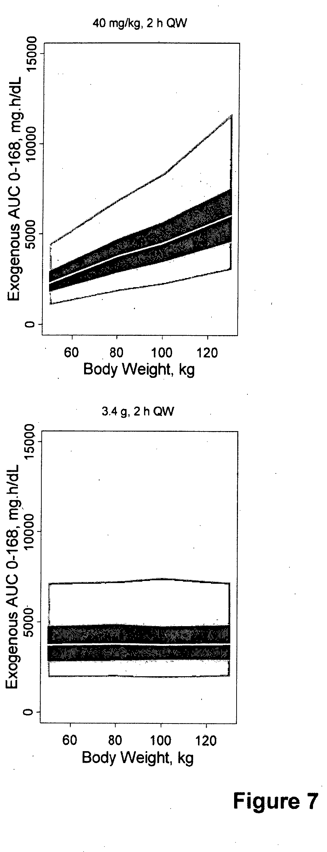

Figure 7 shows the relationship between exogenous ApoA-I AUC₀₋₁₆₈ (for the last dose of weekly regimens infused over 2 h) and body weight. White line represents the median response, dark blue shaded area represents the inter-quartile range and light blue shaded area represents the 95% PI for 100 simulations; and

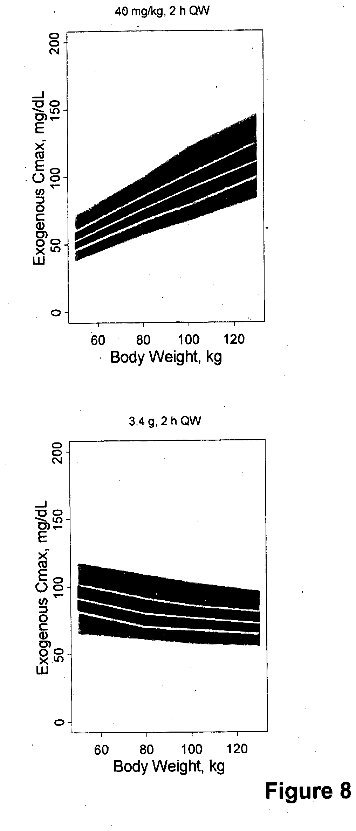

Figure 8 shows the relationship between exogenous ApoA-I Cₘₐₓ (for the last dose of weekly regimens infused over 2 h) and body weight. White line represents the median response, dark blue shaded area represents the inter-quartile range and light blue shaded area represents the 95% PI for 100 simulations.

### DETAILED DESCRIPTION

The invention is defined in the appended claims.

The invention at least partly arises from the unexpected discovery that a fixed dosing regime for apolipoprotein formulations *(e.g.* rHDL) independent of patient body weight has less impact on apoA-1 exposure over a range of patient body weights than that imposed by body weight-adjusted dosing. Thus, there is less inter-patient variability in exposure to apo-AI associated with fixed dosing regimens compared with body weight-adjusted regimes, particularly at the extremes of human patient body weight range.

In one preferred aspect, the invention provides a fixed dosage apolipoprotein formulation at a dosage which is therapeutically effective upon administration to a human of any body weight, or of any body weight in a body weight range.

Disclosed herein, but not claimed, is a method of producing a fixed dosage apolipoprotein formulation including the step of producing the apolipoprotein formulation at a dosage which is therapeutically effective upon administration to a human of any body weight or of any body weight in a body weight range.

It will be appreciated that the present invention relates to apolipoprotein formulations that have therapeutic efficacy in treating one or more diseases, disorders or conditions responsive to therapy by said apolipoprotein formulation.

The apolipoprotein is Apo-A1.

The apolipoprotein formulations of the present invention are formulated with lipid (*e.g.* rHDL).

In a particular embodiment, the apolipoprotein formulation is an rHDL formulation. As used herein, a "reconstituted HDL (rHDL)" formulation may be any artificially-produced apolipoprotein formulation or composition that is functionally similar to, analogous to, corresponds to, or mimics, high density lipoprotein (HDL) typically present in blood plasma. As used herein, an rHDL formulation is not a liposome preparation. rHDL formulations includes within their scope "HDL mimetics" and "synthetic HDL particles". Suitably, the rHDL formulation comprises an apolipoprotein, a lipid and, optionally, a detergent.

Apolipoprotein formulations such as, but not limited to, rHDL formulations may further comprise cholesterol. Apolipoprotein formulations may be produced using organic solvents, which in some cases are used for dissolving the lipid component (e.g. phosphatidylcholine) when producing the formulation, such as described in US patent 5,652,339. However it is preferred that the apolipoprotein formulation is produced in the absence of organic solvent.

The apolipoprotein may be any apolipoprotein which is a functional, biologically active component of naturally-occurring HDL or of a reconstituted high density lipoprotein (rHDL). Typically, the apolipoprotein is either a plasma-derived or recombinant apolipoprotein such as Apo-AI, Apo-AII or Apo-AV, pro-apo-A1 or a variant such as Apo-AI Milano. Preferably, the apolipoprotein is Apo-AI. Also contemplated are biologically-active fragments of the apolipoprotein. Fragments may be naturally occurring, chemical synthetic or recombinant. By way of example only, a biologically-active fragment of Apo-AI preferably has at least 50%, 60%, 70%, 80%, 90% or 95-100% or even greater than 100% of the lecithin-cholesterol acyltransferase (LCAT) stimulatory activity of Apo-AI when formulated in a rHDL preparation.

Suitably, the apolipoprotein is at a concentration of about 5-100 g/L, preferably 10-50g/L or more preferably 25-45g/L . This includes 5, 10, 15, 20, 25, 30, 35, 40, 45, 50, 55, 60, 65, 70, 75, 80, 85, 90, 95 and 100 g/L and any ranges between these amounts. In other embodiments, the apolipoprotein may be at a concentration of from about 5 to 20 g/L, e.g. about 8 to 12 g/L.

The lipid may be any lipid which is a component of naturally-occurring HDL or of reconstituted high density lipoprotein (rHDL). Such lipids include phospholipids, cholesterol, cholesterol-esters, fatty acids and/or triglycerides. Preferably, the lipid is a phospholipid. Non-limiting examples of phospholipids include phosphatidylcholine (PC) (lecithin), phosphatidic acid, phosphatidylethanolamine (PE) (cephalin), phosphatidylglycerol (PG), phosphatidylserine (PS), phosphatidylinositol (PI) and sphingomyelin (SM), sphingosine-1 phosphate or natural or synthetic derivatives thereof. Natural derivatives include egg PC, egg PG, soy bean PC, hydrogenated soy bean PC, soy bean PG, brain PS, sphingolipids, brain SM, galactocerebroside, gangliosides, cerebrosides, cephalin, cardiolipin, and dicetylphosphate. Synthetic derivatives include dipalmitoylphosphatidylcholine (DPPC), didecanoylphosphatidylcholine (DDPC), dierucoylphosphatidylcholine (DEPC), dimyristoylphosphatidylcholine (DMPC), distearoylphosphatidylcholine (DSPC), dilaurylphosphatidylcholine (DLPC), palmitoyloleoylphosphatidylcholine (POPC), palmitoylmyristoylphosphatidylcholine (PMPC), palmitoylstearoylphosphatidylcholine (PSPC), dioleoylphosphatidylcholine (DOPC), dioleoylphosphatidylethanolamine (DOPE), dilauroylphosphatidylglycerol (DLPG), distearoylphosphatidylglycerol (DSPG), dimyristoylphosphatidylglycerol (DMPG), dipalmitoylphosphatidylglycerol (DPPG), distearoylphosphatidylglycerol (DSPG), dioleoylphosphatidylglycerol (DOPG), palmitoyloleoylphosphatidylglycerol (POPG), dimyristoylphosphatidic acid (DMPA), dipalmitoylphosphatidic acid (DPPA), distearoylphosphatidic acid (DSPA), dimyristoylphosphatidylethanolamine (DMPE), dipalmitoylphosphatidylethanolamine (DPPE), dimyristoylphosphatidylserine (DMPS), dipalmitoylphosphatidylserine (DPPS), distearoylphosphatidylethanolamine (DSPE), dioleoylphosphatidylethanolamine (DOPE) dioleoylphosphatidylserine (DOPS), dipalmitoylsphingomyelin (DPSM) and distearoylsphingomyelin (DSSM). The phospholipid can also be a derivative or analogue of any of the above phospholipids.

In other specific embodiments, the lipid is, or comprises, sphingomyelin in combination with a negatively charged phospholipid, such as phosphatidylglycerol (e.g. 1,2-dipalmitoyl-sn-glycero-3-[phospho-rac-(l-glycerol)). A combination of sphingomyelin and phosphatidylglycerol (particularly 1,2-dipalmitoyl-sn-glycero-3-[phospho-rac-(l-glycerol)) is specifically envisaged for use as the lipid. In these embodiments, the sphingomyelin and the phosphatidylglycerol may be present in any suitable ratio, e.g. from 90: 10 to 99:1 (w:w), typically 95:5 to 98:2 and most typically 97:3.

Preferably the phospholipid is, or comprises, phosphatidylcholine, alone or in combination with one or more other phospholipids. An example of another phospholipid is sphingomyelin. The apolipoprotein formulation comprises a detergent wherein the detergent is sodium cholate.

Typically, although not exclusively the lipid may be present at a concentration of 10-100 g/L or preferably 30-60 g/L.

The detergent may be any ionic (e.g cationic, anionic, Zwitterionic) detergent or non-ionic detergent, inclusive of bile acids and salts thereof, suitable for use in apolipoprotein (e.g. rHDL) formulations. Ionic detergents may include bile acids and salts thereof, polysorbates (e.g. PS80), CHAPS, CHAPSO, cetyl trimethyl-ammonium bromide, lauroylsarcosine, tert-octyl phenyl propanesulfonic acid and 4'-amino-7-benzamido-taurocholic acid.

Bile acids are typically dihydroxylated or trihydroxylated steroids with 24 carbons, including cholic acid, deoxycholic acid chenodeoxycholic acid or ursodeoxycholic acid. Preferably, the detergent is a bile salt such as a cholate, deoxycholate, chenodeoxycholate or ursodeoxycholate salt. A particularly preferred detergent is sodium cholate.

As hereinbefore described, the fixed dosage apolipoprotein formulation is at a dosage that is therapeutically effective upon administration to human patients of any body weight or of any body weight in a body weight range. Accordingly, the apolipoprotein formulation dosage is not calculated, determined or selected according to the particular body weight of the human, such as would typically occur with "weight-adjusted dosing".

Rather, the fixed dosage apolipoprotein formulation is determined as a dosage which when administered to human patients of any body weight or of any body weight in a body weight range, would display relatively reduced inter-patient variability in terms of exposure to the apolipoprotein constituents of the apolipoprotein formulation. Relatively reduced inter-patient variability is compared to that observed or associated with weight-adjusted dosing of a patient population.

Variability of exposure may be expressed or measured in terms of the variation in exposure of patients to apolipoprotein following administration of the fixed dosage apolipoprotein formulation. Preferably, the variability is that which would occur when the fixed dosage apolipoprotein formulation is administered to human patients over a weight range compared to the variability that would occur for weight-adjusted dosages administered to human patients over the same weight range as the fixed dosage patients. In some embodiments, exposure to apolipoprotein may be measured as average exposure (e.g. mean or median exposure), total exposure (e.g. amount integrated over time of exposure) or maximum exposure level (e.g. Cmax). Generally, the weight or weight range is 20, 30, 40, 50, 60, 70, 80, 90, 100, 110, 120, 130, 140, 150, 160, 170, 180, 190 or 200 kg, or any range between these values. Preferably, the weight or weight range is 20-200 kg, 20-60 kg, 40-160 kg, 50-80 kg, 60-140 kg, 70-80 kg, 80-120 kg, 100-180 kg or 120-200 kg.

Suitably, the variability is less than 100% or preferably 99%, 98%, 97%, 96% 95%, 94%, 93%, 92%, 91%, or less than 90%, 85% or 80% of the variability that occurs with weight-adjusted dosing. Variability may be calculated and expressed by any statistical representation known in the art, including as a coefficient of variation (e.g. %CV), standard deviation, standard error or the like, although without limitation thereto.

Notwithstanding administration of a fixed dosage apolipoprotein formulation to patients of markedly different body weights, the exposure of the patients to apolipoprotein is surprisingly uniform. Accordingly it is proposed that the therapeutic efficacy of the fixed dosage apolipoprotein formulation will not be substantially compromised or reduced compared to a weight-adjusted dosage.

By way of example only, the present inventors have shown that there is no difference in total exposure to apolipoprotein upon administration of a fixed dosage apolipoprotein formulation to patients in the 60-120 kg weight range. Furthermore, Cₘₐₓ for apolipoprotein decreased by an average of 16% over the 60-120 kg weight range.

In comparison, for weight-adjusted dosing regimes using the same apolipoprotein formulation, a doubling of body weight from 60 kg to 120 kg requires a doubling of the dosage of apolipoprotein and increased apoA-1 exposures.

Fixed dosage apolipoprotein formulations may be administered in multiple doses at any suitable frequency including daily, twice weekly, weekly, fortnightly or monthly. Fixed dosage apolipoprotein formulations may be administered by any route of administration known in the art, such as intravenous administration (*e.g.*, as a bolus or by continuous infusion over a period of time such as over 60, 90, 120 or 180 minutes), by intra-muscular, intra-peritoneal, intra-arterial including directly into coronary arteries, intra-cerebrospinal, sub-cutaneous, intra-articular, intra-synovial, intra-thecal, oral, topical, or inhalation routes. Typically, fixed dosage apolipoprotein formulations are administered parenterally, such as by intravenous infusion or injection.

Preferred fixed dosages include 0.1-15g, 0.5-12g, 1-10g, 2-9g, 3-8g, 4-7g or 5-6g of apolipoprotein. Particularly preferred fixed dosages include 1-2g, 3-4g, 5-6g or 6-7g of apolipoprotein. Non-limiting examples of specific fixed dosages include 0.25g, 0.5g, 1g, 1.7g, 2g, 3.4g, 4g, 5.1g, 6g, 6.8g and 8g of apolipoprotein.

Non-limiting, specific examples of fixed dosage administration regimes that may be employed include 0.25g, 0.5g, 1g, 1.7g, 2g, 3.4g, 4g, 5.1g, 6g, 6.8g or 8g weekly by intravenous infusion over 90 min, 0.25g, 0.5g, 1g, 1.7g, 2g, 3.4g, 4g, 5.1g, 6g, 6.8g or 8g apolipoprotein weekly by intravenous infusion over 120 min or 0.25g, 0.5g, 1g, 1.7g, 2g, 3.4g, 4g, 5.1g, 6g, 6.8g or 8g apolipoprotein twice weekly by intravenous infusion over 90 min or over 120 min.

In yet another aspect, the invention provides a method of prophylactically or therapeutically treating a disease, disorder or condition in a human including the step of administering to the human an apolipoprotein formulation disclosed herein.

Suitably, the disease, disorder or condition responsive to prophylactic or therapeutic administration of the fixed dosage apolipoprotein formulation includes such diseases, disorders or conditions as cardiovascular disease (e.g. acute coronary syndrome (ACS), atherosclerosis, unstable angina pectoris and myocardial infarction) or diseases, disorders or conditions such as diabetes, stroke, impaired renal function, or myocardial infarction that predispose to ACS, hypercholesterolaemia (*e.g.* elevated serum cholesterol or elevated LDL cholesterol) and hypocholesterolaemia resulting from reduced levels of high-density lipoprotein (HDL), such as is symptomatic of Tangier disease, although without limitation thereto.

A fixed dosage, apolipoprotein kit comprising one or more unit doses of the fixed dosage apolipoprotein formulation and one or more other kit components is disclosed but not claimed.

Suitably, the kit is for prophylactically or therapeutically treating a disease, disorder or condition in a human, as hereinbefore described.

Non-limiting examples of one or more other kit components include instructions for use; vials, containers or other storage vessels containing each of the unit doses; delivery devices such as needles, catheters, syringes, tubing and the like; and/or packaging suitable for safely and conveniently storing and/or transporting the kit. Preferably the instructions for use are a label or package insert, wherein the label or package insert indicates that the apolipoprotein formulation may be used to treat a disease or condition such as cardiovascular disease by administering a fixed dose amount to a human subject in need thereof.

A 'package insert' refers to instructions included in commercial packages of the apolipoprotein formulations, that contains information about the indications, usage, dosage, administration, contraindications and/or warnings concerning the use of such apolipoprotein formations.

For the purposes herein, a 'vial' refers to a container which holds an apolipoprotein formulation. The vial may be sealed by a stopper pierceable by a syringe, Generally, the vial is formed from a glass material. Accordingly, a vial preferably comprises the lyophilized rHDL formulation with a protein content of 0.25g, 0.5g, 1g, 2g, 2.5g, 3g, 3.5g, 4g, 4.5g, 5g, 5.5g, 6g, 6.5g, 7g, 8g or 10 g per vial. More preferably the apolipoprotein content is either 0.5g, 1g, 2g, 4g, 6g, 8g or 10 g per vial.

The apolipoprotein formulation in the vial can be in various states including liquid, lyophilized, frozen *etc.* The fixed dosage apolipoprotein formulation is preferably stable as a liquid. Stability may be measured by any means known in the art, although turbidity is a preferred measure. A turbidity level of below about 10, 15, 20, or 30 NTU can generally be considered a stable fixed dosage apolipoprotein formulation. Turbidity measurements can be taken by incubating the fixed dosage apolipoprotein formulations over time periods such as 0 hr, 2 hr, 4hr, 6 hr, 12 hr, 18 hr, 24 hr, 36 hr, 72 hr, 7 days and 14 days at storage temperatures such as room temperature or 37°C. Preferably the fixed dosage apolipoprotein formulation is considered to be stable as a liquid when it is stored for 14 days at room temperature and exhibits a turbidity of less than about 15 NTU.

The kit may facilitate administration of the fixed dosage apolipoprotein formulation by a health professional or self-administration by a patient or caregiver.

As previously described, the fixed dosage apolipoprotein formulation disclosed herein may be any apolipoprotein formulation. Preferably, the apolipoprotein formulation is an rHDL formulation that comprises an apolipoprotein, lipid and, optionally, a detergent. Accordingly, the fixed dosage apolipoprotein or rHDL formulation may generally apply to any such formulation that would usually be administered by "weight-adjusted" dosing. Non-limiting examples of rHDL formulations include those described in WO2003/096983; US20040266662; WO2005/041866; WO2006/100567; WO06/20069240; and WO2010/093918, WO2012/000048 and WO2012/109162.

One particular non-limiting example is an rHDL formulation comprising Apo-A1 and one or more phospholipids including sphingomyelin and one or more negatively charged phospholipids such as phosphatidylinositol and phosphatidylglycerol.

In some embodiments, detergent is absent from the fixed dosage rHDL formulation. In other embodiments, detergent is present in the fixed dosage rHDL formulation. In one preferred embodiment, the fixed dosage rHDL formulation comprises a detergent at a level which is not toxic, or at least displays relatively low toxicity. In this regard, reference is made to WO2012/000048 which provides a detailed description on an rHDL formulation according to this embodiment.

In work leading to the present invention, it had been shown that some rHDL formulations displayed liver toxicity upon administration of the rHDL formulation to a human, such as evidenced by abnormal or compromised liver function. Non-limiting examples of liver function(s) that may be abnormal or compromised include elevated alanine aminotransferase activity (ALT), elevated aspartate aminotransferase (AST) activity and/or elevated bilirubin levels. An example of an rHDL formulation at a level that caused liver toxicity is described in Tardif et al., 2007, JAMA-Exp. 297 E1. Preferably the fixed dosage apolipoprotein formulation does not display liver toxicity.

Liver toxicity can be evaluated by various *in vitro* and *in vivo* models. One example of an *in vitro* model uses HEP-G2 cells. This involves growing HEP-G2 cells into the log phase. The cells are then removed from the culture medium and washed in PBS prior to trypsinization and resuspension in 10 mL of culture medium (90 % DMEM, 10 % inactivated FCS, 1 % nonessential amino acids, 1 % Pen/Strep). Cell growth and viability are monitored using a Neubauer haemocytometer and trypan blue staining. Aliquots of 100 µL containing 10×10⁴ C/mL are subsequently seeded, into 96 well F-bottom plates and incubated overnight at 37°C, 5% CO₂, 95% H₂O. Samples (700 µL) containing the test articles (*e.g* rHDL formulations) are prepared by addition of culture medium. The medium from the first row of wells is removed and 200 µL of the test article solution added. A serial 1:2 dilution series is completed on the plates. The plates are then incubated for 72 hours at 37°C, 5% CO₂, 95% H₂O. After which the cell viability is determined. This can be done by adding 50 µL of 3x Neutral Red Solution (70mg Neutral Red in 100mL PBS) to each well. The plates are incubated for 2 hours at 37°C, 5% CO₂, 95% H₂O and the wells washed once with 200 µL PBS. After this 100 µL of ethanol is added to each plate and the plates shaken for 20 minutes prior to being read at 540nm.

An example of an *in vivo* hepatoxicity model is the conscious rabbit model. The model uses rabbits which have been placed in a restraint device (rabbit holder) and i.v. catheters inserted into their ear veins. Test articles are given as a 40 minute i.v. infusion. Blood samples are taken from the ear artery and collected into serum and streptokinase-plasma (5%) vials. Blood samples are processed to serum, stored at -20°C and to plasma and stored at - 80°C. Samples can then be assessed for the level of ALT and AST activity using enzymatic photometric test kits available commercially (Greiner Biochemica). Whilst human Apo A-I levels can be determined using a nephelometric assay. Preferably, the level of detergent when present in the fixed dosage apolipoprotein formulations is about 5-35% of that which displays liver toxicity. This range includes, for example, 5%, 10%, 15%, 20%, 25%, 30% and 35%. More preferably, the level of detergent is about 5-20% of that which displays liver toxicity. Advantageously, the level is about 5-10% of that which displays liver toxicity. Preferably, these levels are expressed in terms of the minimum or threshold level of detergent that displays liver toxicity.

By way of example, a level of detergent which has been shown to cause, result in or at least be associated with liver toxicity is 0.3g/g Apo-AI or 6g/L rHDL formulation (at 20g/L Apo-AI). Accordingly, 5-10% of this level of detergent is 0.015-0.03 g/g Apo-AI or 0.5-0.9 g/L rHDL formulation (at 30g/L Apo-AI).

The "level" of detergent may be an absolute amount of detergent, a concentration of detergent (e.g. mass per unit volume of rHDL formulation) and/or a ratio of the amount or concentration of detergent relative to another amount or concentration of a component of the rHDL formulation. By way of example only, the level of detergent may be expressed in terms of the total mass of apolipoprotein (e.g. Apo-AI) present in the rHDL formulation.

A detergent concentration no less than about 0.45 g/L of rHDL formulation with 30 g/L apolipoprotein is optimal in terms of both stability and non-toxicity. Stability may advantageously be measured by any means known in the art, although turbidity of the rHDL formulation is a preferred measure.

In a further preferred embodiment, the fixed dosage rHDL formulation comprises a lipid at a level which does not cause liver toxicity. Suitably, the level of lipid is about 20-70% of that which causes, or is associated with, liver toxicity. In particular embodiments, the level of lipid is preferably about 25%, 30%, 35%, 40%, 45%, 50%, 55%, 60% or 65% of that which causes, or is associated with, liver toxicity, and any ranges between these amounts. Preferably, these levels are expressed in terms of the minimum or threshold level of lipid that displays liver toxicity.

By way of example, a level of lipid which has been shown in work leading to the present invention to cause, result in or at least be associated with liver toxicity is 84 g/L. Accordingly the lipid is preferably at a concentration of about 30-60 g/L. This includes 30, 35, 40, 45, 50, 55 and 60 g/L and any ranges between these amounts. A particularly advantageous concentration of lipid is about 30-50g/L, or in certain embodiments about 34 or 47g/L.

The "level" of lipid may be an absolute amount of lipid, a concentration of lipid (e.g. mass per unit volume of rHDL formulation) and/or a ratio of the amount or concentration of lipid relative to another amount or concentration of a component of the fixed dosage apolipoprotein formulation. By way of example only, the level of lipid may be expressed in terms of a molar ratio of apolipoprotein (*e.g*. Apo-AI) present in the fixed dosage rHDL formulation.

In one preferred embodiment, the molar ratio of apolipoprotein: lipid is in the range 1:20 to 1:100. This range includes molar ratios such as 1:30, 1:40, 1:50, 1:60, 1:70, 1:80 and 1:90. More preferably, the molar ratio of apolipoprotein:lipid is in the range of 1:40 to 1:75. A particularly advantageous ratio of apolipoprotein:lipid is about 1:40 or 1:55.

In other embodiments, the molar ratio of apolipoprotein:lipid is in the range from about 1:80 to about 1:120. For example, the ratio may be from 1:100 to 1:115, or from 1:105 to 1:110. In these embodiments, the molar ratio may be for example from 1:80 to 1:90, from 1:90 to 1:100, or from 1:100 to 1:110.

Suitably, the fixed dosage apolipoprotein formulation disclosed herein further comprises a stabilizer. In particular, the stabilizer maintains stability of the rHDL formulation during lyophilisation. Suitably the stabilizer is a carbohydrate such as a sugar or sugar alcohol. Examples of suitable sugar or sugar alcohols are fructose, trehalose, mannitol and sorbitol. Preferably, the stabilizer is a disaccharide sugar such as sucrose. A preferred concentration of sucrose is about 10-85 g/L (equivalent to about 1.0-8.5% w/v) of fixed dosage apolipoprotein formulation. Preferably, the concentration of sucrose is about 46-48 g/L (equivalent to about 4.6-4.8% w/v) or about 75 g/L (equivalent to about 7.5% w/v), which is relatively reduced compared to rHDL formulations such as CSL111. It is proposed that this relatively reduced sucrose may allow for a faster infusion rate of the rHDL formulation of the invention. Other stabilizers may be or include amino acids (e.g. glycine, proline), antioxidants, emulsifiers, surfactants, chelating agents, gelatine, synthetic oils, polyols, alginate or any pharmaceutically acceptable carriers and/or excipients, although without limitation thereto. In this regard, reference is made by way of example to "Pharmaceutical Formulation Development of Peptides and Proteins", Frokjaer et al., Taylor &; Francis (2000), "Handbook of Pharmaceutical Excipients", 3rd edition, Kibbe et al., Pharmaceutical Press (2000) and International Publication WO2009/025754.

Reduction or removal of detergent may be performed by any means known in the art including filtration, hydrophobic adsorption or hydrophobic interaction chromatography, dialysis, ion-exchange adsorption and ion-exchange chromatography, for example.

In some embodiments, non-polar polystyrene resins may be suitable for reducing detergent levels. Such resins preferably are in the form of a cross-linked copolymer (e.g. a cross-linked styrene and divinylbenzene copolymer). Non-limiting examples include Amberlite XAD-2 and Bio Beads SM.

Filtration includes gel filtration, gel permeation, diafiltration and ultrafiltration, although without limitation thereto, as are well understood in the art. A non-limiting example of gel permeation may utilize porous, cross-linked dextran such as Sephadex resins.

In a particularly preferred embodiment particularly suitable for large scale manufacture, the detergent level is reduced by diafiltration.

So that preferred embodiments of the invention may be fully understood and put into practical effect, reference is made to the following non-limiting Examples.

### EXAMPLES

### INTRODUCTION

Reconstituted high density lipoprotein (HDL) consists of purified human apolipoprotein A-I (ApoA-I), the primary protein constituent of HDL. Low plasma HDL is associated with an increased risk of coronary artery disease, one of the principal causes of death in developed countries. Consequently, it has been postulated that HDL may be protective of atherosclerosis. The mechanism by which HDL exerts its atheroprotective effect is complex and incompletely understood, but is believed to act at various steps involved in the efflux of cholesterol from peripheral cells (including arterial cells) to the liver.

The rHDL formulation described in this example is a novel therapeutic agent that mimics endogenous HDL. Proof of concept was demonstrated for an older formulation of reconstituted HDL, CSL111, in the ERASE trial, a Phase 2a study of short term CSL111 infusions in 183 patients. However, development of CSL111 was ceased due to hepatotoxicity (Tardiff *et al*., 2010, *supra* and also described in more detail in WO2012/000048).

The rHDL formulation described in this example consists of purified human apolipoprotein A-1 (ApoA-I), the primary protein constituent of HDL (as hereinbefore described and also described in more detail in WO2012/000048).

The primary objective was to provide a justification for an appropriate dosing strategy (weight-adjusted or fixed dosing) for the rHDL formulation using a model-based approach and Monte Carlo simulations.

### METHODS

### General Approach

Monte Carlo simulations were performed using interim population PK models for ApoA-I developed in this analysis. The simulations used all components of the pharmacokinetic (PK) model including important covariates determined to influence ApoA-I disposition. The estimated population means (typical values) and intersubject variability values were used to define distributions for the interim PK parameters and simulations used random sampling from those distributions. The randomness described by this sampling process reflected the intersubject variability in the model parameters.

The randomly generated PK parameters were used to simulate plasma concentrations of ApoA-I for each virtual patient in the simulations. Estimates of residual error, from the PK model, were used to introduce measurement error into the concentration predictions. Using the concentration predictions (that now incorporated both intersubject variability and measurement error), selected ApoA-I exposure metrics were calculated for each virtual patient.

Results are summarized graphically and in tabular form.

### Simulation Specifications

ApoA-I exposure was simulated separately for each of the following dosing regimens:
- 40, 70 and 100 mg/kg weekly by intravenous infusion (IVI) over 90 min and 120 min
- 1.7, 3.4 and 5.1 g weekly by IVI over 90 min and 1.7, 3.4, 5.1 and 6.8 g weekly by IVI over 120 min
- 1.7, 3.4 and 5.1 g twice weekly by IVI over 90 min and over 120 min

Dosing was assumed to be continued for a period of 4 weeks. For the twice weekly regimens, the second weekly dose was administered 72 hours after the start of the first dose administration.

Since body weight, creatinine clearance (CLCR) and sex were identified as significant sources of intersubject variability for ApoA-I, clinically relevant ranges of these patient covariates were included in the simulations:
- Body weight: 60, 80, 100 and 120 kg for ApoA-I
- CLCR: 50, 80 and 140 ml/min
- Age: 20, 60 and 80 yrs
- Males and females in equal proportions.

Each simulation set included each relevant patient covariate and dosing regimen combination. Therefore, there were 456 virtual subjects (i.e 4 body weight values x 3 CLCR values x 2 sex x 19 dosing regimens) in the ApoA-I simulation set. 100 simulations were performed.

Plasma concentration measurements were assigned to occur at 0, 1, 2, 4, 8, 12, 24, 48, 72, 96, 120 and 168 h from the start of the first dose for the weekly regimens and at 0, 1, 1.5, 2, 3, 4, 6, 8, 12, 18, 24, 36, 48, 72, 73, 73.5, 74, 75, 76, 78, 80, 90, 96, 108, 120, 144 and 168 h from the start of the first dose for the twice weekly regimens. Sampling was conducted during the first and fourth weeks of dosing.

Endpoints of interest were total plasma concentration vs. time profiles and exogenous maximum plasma concentration (Cₘₐₓ), area under the curve from 0 to 72 hr (AUC₀₋₇₂) and area under the curve from 0 to 168 hr (AUC₀₋₁₆₈) following each dosing regimen. Cmax and AUC₀₋₇₂ were calculated for the first and last doses while AUC₀₋₁₆₈ was calculated for the first and last weeks of dosing. Exogenous plasma concentrations for exposure determinations were calculated by subtracting the simulated baseline plasma concentration from the simulated total plasma concentration at each time point.

### RESULTS

### Projected ApoA-I Exposures by Dosing Regimen

Projected median (FIG. 1) and distributions (FIG. 2) of ApoA-1 plasma concentration vs. time profiles are shown by dosing regimen (2 h infusion durations shown). Given a median body weight of 90 kg, median exposure for the 40 mg/kg doses reflects a 3.6 g dose and is comparable to the 3.4 g fixed dose (see FIG. 1). Similarly, median exposure for the 70 mg/kg dose reflects 6.3 g and is approximately comparable to the 6.8 g fixed dose.

Table 1 summarizes projected ApoA-1 exposures during the fourth week of dose administration by dosing regimen. Despite similar mean exposures for the 40 mg/kg and 3.4 g dosing regimens and for the 70 mg/kg and 6.8 g dosing regimens, there was approximately 8-10% greater variability (as measured by %CV in exposure metrics) associated with the weight-adjusted dosing regimens compared with the fixed dosing weekly regimens, regardless of infusion duration. FIGS 3-5 show modestly broader exposure bands for the 40 mg/kg and 70 mg/kg weight-adjusted dosing regimens relative to the equivalent fixed doses (3.6 g and 6.3g, respectively) infused over 2 hours weekly.

### Effect of Body Weight on ApoA-I Exposures by Dosing Regimen

For fixed dosing regimens, body weight did not influence total exposure (AUC) while Cₘₐₓ decreased by an average of 16% with a doubling of body weight from 60 kg to 120 kg (FIG. 6 to FIG. 8; right panel). In comparison, for weight-adjusted dosing regimens, a doubling of body weight from 60 kg to 120 kg results in a doubling of dose and, consistent with linear pharmacokinetic theory, exposure is projected to increase accordingly (FIG. 6 to FIG. 8; left panel). Thus, body weight had a smaller impact on ApoA-I exposure than that imposed by body weight-adjusted dosing which was more evident at the extremes of the body weight range.

### DISCUSSION

The results of the simulation analysis suggest that body weight had a smaller impact on ApoA-I exposures than that imposed by body weight-adjusted dosing. Thus, there is expected to be less variability in exposures associated with fixed dosing regimens compared with body weight-adjusted regimens at the extremes of the body weight range.

The modeling and simulation results described herein support the administration of fixed dosing regimens of the rHDL formulation in order to reduce variability in ApoA-I exposures over a broad range of body weights.

Throughout the specification, the aim has been to describe the preferred embodiments of the invention without limiting the invention to any one embodiment or specific collection of features. Various changes and modifications may be made to the embodiments described and illustrated without departing from the present invention.

**Table 1: Projected exogenous ApoA-I exposures by dosing regimen in week 4.**

| Dose | Dose Unit | Infusion Duration (h) | Frequency | AUC₀₋₇₂ (mg.h/dL) | AUC₀₋₁₆₈ (mg.h/dL) | Cmax (mg/dl) | Accumulation ratio based on first:last | | |
|---|---|---|---|---|---|---|---|---|---|
| | | | | | | | AUC₀₋₇₂ | AUC₀₋₁₆₈ | Cmax |
| 1.7 | g | 2 | QW | 1437(24) | 1952.4(34.3) | 38.8(15.7) | 1.25 | 1.24 | 1.16 |
| 3.4 | g | 2 | QW | 2732.9(24.9) | 3787.5(35.1) | 791(16) | 120 | 1.20 | 113 |
| 5.1 | g | 2 | QW | 4102.5(25.2) | 5804.3(35.1) | 120.4(16.4) | 1.20 | 1.20 | 113 |
| 6.8 | g | 2 | QW | 5371.8(25.6) | 7608.1(35.6) | 159.7(16.2) | 1.17 | 1.17 | 1.11 |
| 40 | mg/kg | 2 | QW | 2859.4(34.1) | 3977.5(43.7) | 82.2(25.1) | 120 | 1.20 | 1.14 |
| 70 | mg/kg | 2 | QW | 4845.1(34) | 6770.2(43.1) | 144.1(24.7) | 1.17 | 1.16 | 1.11 |
| 100 | mg/kg | 2 | QW | 6852.7(35.1) | 9638.6(44.6) | 207.4(24.9) | 1.15 | 1.15 | 110 |
| 1.7 | g | 1.5 | QW | 1423.2(24.9) | 1891.6(35.4) | 40.1(16) | 1.24 | 1.19 | 1.15 |
| 3.4 | g | 1.5 | QW | 2757(24.2) | 3840.9(33.9) | 81.5(16.3) | 1.19 | 1.20 | 1.12 |
| 5.1 | g | 1.5 | QW | 4023.4(25.4) | 5672.1(35.7) | 123.2(15.9) | 1.17 | 1.18 | 112 |
| 40 | mg/kg | 1.5 | QW | 2810.9(33.4) | 3896.2(42.8) | 84.6(24.1) | 1.18 | 1.19 | 1.13 |
| 70 | mg/kg | 1.5 | QW | 4900.7(34.2) | 6910.7(43.2) | 148.3(24.4) | 1.18 | 1.17 | 112 |
| 100 | mg/kg | 1.5 | QW | 6937.5(34.9) | 9743.4(44.6) | 214.8(24.1) | 1.16 | 1.16 | 1.11 |
| 1.7 | g | 2 | BW | 1845.9(33.4) | 3885.1(35) | 47.3(19.9) | 1.60 | 1.33 | 1.42 |
| 3.4 | g | 2 | BW | 3575.7(34) | 7498.4(35.8) | 96.8(19.4) | 1.57 | 1.28 | 139 |
| 5.1 | g | 2 | BW | 5320.6(33.5) | 11127.8(35.3) | 147.2(19.1) | 1.55 | 1.25 | 1.37 |
| 1.7 | g | 1.5 | BW | 1845.9(33.4) | 3884.6(35) | 48.6(19.6) | 1.60 | 1.33 | 141 |
| 3.4 | g | 1.5 | BW | 3575.8(34) | 7497.7(35.8) | 99.3(19.1) | 1.56 | 1.28 | 1.38 |
| 5.1 | g | 1.5 | BW | 5320.9(33.5) | 11126.6(35.3) | 150.4(18.7) | 1.54 | 1.25 | 1.36 |

| | | | | | | | | | |
|---|---|---|---|---|---|---|---|---|---|
| Values are mean (%CV) and reflect a typical subject (body weight = 90 kg, CLCR = 80 ml/min) and averaged across males and females. QW = weekly dosing, BW = twice weekly dosing | | | | | | | | | |

## Claims

1. A fixed dosage reconstituted high density lipoprotein (rHDL) formulation comprising Apo-A1, a lipid and a detergent, for use in prophylactically or therapeutically treating a disease, disorder or condition in a human selected from the group of cardiovascular disease, hypercholesterolaemia or hypocholesterolaemia, acute coronary syndrome (ACS), atherosclerosis, unstable angina pectoris, and myocardial infarction wherein the rHDL formulation is therapeutically effective upon administration to a human of any body weight in the body weight range 60-120 kg, wherein the fixed dosage comprises an amount of Apo-AI in a range selected from: 5-6g and 6-7g of apolipoprotein, and the Apo-AI is purified from plasma, the lipid is phosphatidylcholine, and the detergent is sodium cholate, the ratio between the apolipoprotein and the lipid is from 1:40 to 1:75 (mol:mol), and the sodium cholate is present at a concentration of 0.5 to 0.9 g/L.

2. The fixed dosage rHDL formulation of claim 1 wherein the fixed dosage comprises an amount of Apo-Al selected from 5.1g, 6g or 6.8g apolipoprotein.

3. The fixed dosage rHDL formulation for use according to claim 1, wherein the fixed dosage apolipoprotein formulation is administered by intravenous infusion or injection.

4. The fixed dosage rHDL formulation for use according to claim 1, wherein the fixed dosage apolipoprotein is administered weekly.

## Patentansprüche

1. Rekonstituiertes-High-Density-Lipoprotein(rHDL)-Formulierung mit festgelegter Dosis, umfassend Apo-A1, ein Lipid und ein Detergens, zur Verwendung bei der prophylaktischen oder therapeutischen Behandlung einer Krankheit, einer Störung oder eines Leidens beim Menschen, ausgewählt aus der Gruppe Herz-Kreislauf-Krankheit, Hypercholesterinämie oder Hypocholesterinämie, akutem Koronarsyndrom (ACS), Atherosklerose, instabiler Angina pectoris und Myokardinfarkt, wobei die rHDL-Formulierung bei Verabreichung an einen Menschen mit einem Körpergewicht im Körpergewichtsbereich 60-120 kg therapeutisch wirksam ist, wobei die festgelegte Dosis eine Menge von Apo-AI in einem Bereich ausgewählt aus 5-6 g und 6-7 g Apolipoprotein umfasst und das Apo-AI aus Plasma aufgereinigt ist, es sich bei dem Lipid um Phosphatidylcholin und bei dem Detergens um Natriumcholat handelt, das Verhältnis zwischen dem Apolipoprotein und dem Lipid 1:40 bis 1:75 (mol:mol) beträgt und das Natriumcholat in einer Konzentration von 0,5 bis 0,9 g/l vorliegt.

2. rHDL-Formulierung mit festgelegter Dosis nach Anspruch 1, wobei die festgelegte Dosis eine Menge von Apo-AI ausgewählt aus 5,1 g, 6 g oder 6,8 g Apolipoprotein umfasst.

3. rHDL-Formulierung mit festgelegter Dosis zur Verwendung gemäß Anspruch 1, wobei die Apolipoproteinformulierung mit festgelegter Dosis über intravenöse Infusion oder Injektion verabreicht wird.

4. rHDL-Formulierung mit festgelegter Dosis zur Verwendung gemäß Anspruch 1, wobei das Apolipoprotein mit festgelegter Dosis wöchentlich verabreicht wird.

## Revendications

1. Formulation de lipoprotéines à haute densité reconstituée (rHDL) à dosage fixe comprenant Apo-A1, un lipide et un détergent, pour une utilisation dans le traitement de manière prophylactique ou de manière thérapeutique d'une maladie, d'un trouble ou d'une affection chez un humain choisi(e) dans le groupe composé d'une maladie cardiovasculaire, de l'hypercholestérolémie ou de l'hypocholestérolémie, du syndrome coronarien aigu (ACS), de l'athérosclérose, de l'angine de poitrine instable et d'un infarctus du myocarde, la formulation de rHDL étant thérapeutiquement efficace lors d'une administration à un humain d'un quelconque poids corporel dans la plage de poids corporel de 60 à 120 kg, le dosage fixe comprenant une quantité d'Apo-AI dans une plage choisie parmi : 5 à 6 g et 6 à 7 g d'apolipoprotéine, et l'Apo-AI étant purifiée à partir d'un plasma, le lipide étant la phosphatidylcholine, et le détergent étant le cholate de sodium, le rapport entre l'apolipoprotéine et le lipide étant de 1 : 40 à 1 : 75 (moles : moles), et le cholate de sodium étant présent en une concentration de 0,5 à 0,9 g/L.

2. Formulation de rHDL à dosage fixe selon la revendication 1, le dosage fixe comprenant une quantité d'Apo-AI choisie parmi 5,1 g, 6 g ou 6,8 g d'apolipoprotéine.

3. Formulation de rHDL à dosage fixe pour une utilisation selon la revendication 1, la formulation d'apolipoprotéine à dosage fixe étant administrée par perfusion ou injection intraveineuse.

4. Formulation de rHDL à dosage fixe pour une utilisation selon la revendication 1, l'apolipoprotéine à dosage fixe étant administrée de manière hebdomadaire.
